# EUROPEAN PATENT SPECIFICATION

(11) **EP 2 738 248 B1**
(45) Date of publication and mention of the grant of the patent: **15.06.2016**
(21) Application number: 12864984.5
(22) Date of filing: 07.03.2012
(51) Int. Cl.: C12N 5/00, C12N 5/071, C12N 11/04, C12M 1/32, C12M 1/00, C12M 3/00, G01N 33/50, A61L 27/54, A61L 27/52, A61L 27/38

(54) **METHOD FOR MANUFACTURE IN VITRO VASCULARIZED TISSUE**
VERFAHREN ZUR HERSTELLUNG VON IN VITRO VASKULARISIERTEM GEWEBE
PROCÉDÉ D'ÉLABORATION D'UN TISSU VASCULARISÉ IN VITRO

(30) Priority: 11.01.2012 KR 20120003566
(43) Date of publication of application: 04.06.2014
(73) Proprietor: Kang, Gook-Jin, Seoul 138-773 (KR)
(72) Inventor: Kang, Gook-Jin, Seoul 138-773 (KR)
(74) Representative: Rentsch Partner AG
(86) International application number: PCT/KR2012/001667
(87) International publication number: WO 2013/105695

(56) References cited:
- KR-B1- 100 921 487
- NICHOL J W ET AL: "Cell-laden microengineered gelatin methacrylate hydrogels", BIOMATERIALS, ELSEVIER SCIENCE PUBLISHERS BV., BARKING, GB, vol. 31, no. 21, 1 July 2010 (2010-07-01), pages 5536-5544, XP002725666, ISSN: 0142-9612, DOI: 10.1016/J.BIOMATERIALS.2010.03.064 [retrieved on 2010-04-24]
- TSANG ET AL.: 'Three-dimensional tissue fabrication' ADVANCED DRUG DELIVERY REVIEWS vol. 56, 19 July 2004, pages 1635 - 1647, XP004550361
- MOON ET AL.: 'Biomimetic hydrogels with pro-angiogenic properties' BIOMATERIALS vol. 31, 24 February 2010, pages 3840 - 3847, XP026947557
- SAIK ET AL.: 'Biomimetic hydrogels with immobilized EphrinAl for therapeutic angiogenesis' BIOMACROMOLECULES vol. 12, 06 June 2011, pages 2715 - 2722, XP055081312

## Description

### [Technical Field]

The present invention relates to a method for manufacturing *in vitro* vascularized tissues, and more particularly, to a method for manufacturing an *in vitro* vascularized tissue that may derive angiogenesis *in vitro* from a corresponding tissue using vascular cells, thus obtaining the *in vitro* vascularized tissue, so as to use the same in *in vitro* research of diseases in regard to vascularized tissues and development of a treatment method thereof.

### [Background Art]

In recent years, as cancer incidence has increased due to aging populations, increases in smoking, changes in dietary patterns, lack of exercise, or the like, development of anticancer medicines for treating cancer has also actively progressed. Anticancer medicines developed in the art have advantageous effects but mostly involve some problems such as toxicity caused by destruction of normal cells, drug tolerance to anticancer medicines, cancer recurrence, or the like, therefore, an improved anticancer medicine based on a novel mechanism is still required. Accordingly, there have been intensive efforts to discover the causes of the occurrence of cancers and progress thereof thus propose that more fundamental solutions to overcome these diseases are required and, in 1971, J. Folkman suggested an anti-angiogenic agent, as a novel alternative idea, in order to prevent growth and metastasis of tumors (see Folkman et al., N. Engl. J. Med., 285, p1182, 1971).

Angiogenesis refers to a process of generating new blood vessels from existing blood vessels, and becomes a cause of various diseases including, for example, metastasis of tumors, diabetic retinopathy, psoriasis, chronic inflammation, ulcers, or the like [Carmeliet et al., Nature, 407, p249, 2000]. In particular, in a case of serious angiogenesis such as that associated with cancer, growth and metastasis of tumors can be efficiently inhibited by simply suppressing such angiogenesis.

Targets for developing an anticancer medicine by suppressing angiogenesis may include, for example, a vascular endothelial growth factor (VEGE), vascular endothelial growth factor receptor (VEGER), basic fibroblast growth factor (bFGF), transfer growth factor alpha and beta (TGF-α, -β), epidermal growth factor (EGF), platelet-derived growth factor (PDGF), or the like.

When administering the foregoing anticancer medicine to a patient, effective anticancer agents vary in term of kinds and dosages depending upon patients thereof, therefore, it is not appropriate to connect the same to data known in academic art or experience of physicians. Further, since such anticancer medicine typically destroys normal cells as well as cancer cells, the number of times anticancer medicine is administered to a patient is limited to 4 times at most, thereby making it impossible to test a variety of kinds or dosages of anticancer medicine.

Accordingly, although anticancer medicine must be directly applied to particular cancer cells in the patient for examination, in order to discover any suitable anticancer medicine, a solution to overcome the above problem has still not been proposed.

Therefore, selection of such anticancer medicines which have such a low success rate is nothing but a probability game involving the life of cancer patients whose days are numbered. However, cancer patients typically have no grounds with which to make a selection except for relying on an uncertain treatment method and adhering to the selection of a physician. Further, despite that the expenses to the patient or his/her family are considerable, the results of the treatment are far from certain.

Further, with regard to selection of appropriate methods for treatment of diseases in vascularized tissues such as the liver, brain, or the like, there is no choice but to rely on the experience of physicians or data known in academic art among a number of treatment methods such as cancer treatment.

In addition, recent radiation methods for cancer treatment widely used in the art may also entail the foregoing problems. Radiation-based cancer treatment may irradiate both of normal cells and cancer cells with radioactive rays whereby the normal cells then rapidly recover, as compared to the cancer cells, wherein the radioactive rays for treatment may include, for example, X-rays, gamma-rays, electron rays, neutron rays, proton rays, baryon rays, or the like, in a range of 1,000,000 volts or more, and a type or dosage of radiation must be different according to patients or body parts of the patient irradiated by the radioactive rays.

That is, although a position of a cancer cell can be identified through CT or MRI, a correct dosage of radiation is actually determined only by the experience of a skilled person. Accordingly, there are still problems in that cancer cells are not destroyed due to an insufficient dosage of radiation, or normal cells are destroyed due to an excessive dosage of radiation, hence potentially causing a patient fatal damage.

### [Disclosure]

### [Technical Problem]

Accordingly, in order to solve the above-mentioned problems, an object of the present invention is to provide a method for manufacturing *in vitro* vascularized tissues, that uses vascular cells *in vitro* to derive angiogenesis from corresponding tissues, thus obtaining the *in vitro* vascularized tissues, so as to use the same in *in vitro* research of diseases in regard to the vascularized tissues and development of a treatment method thereof.

### [Technical Solution]

In order to accomplish the foregoing object, according to the present invention, there is provided a method for manufacturing *in vitro* vascularized tissues, which includes: supplying vascularized tissue cells in a container; supplying a hydrogel to cover the vascularized tissue cells; shaping the hydrogel to form a supply hole separated from the vascularized tissue cells on an upper side of the hydrogel; and supplying vascular cells in the supply hole.

The supply hole may be formed by a molding cover to press the hydrogel and shape the same. Therefore, a shape and size of the supply hole may be freely altered depending upon the molding cover.

Further, the vascularized tissue cells may be at least one selected from cancer cells, brain cells or liver cells. That is, any cell enabling angiogenesis through signal exchange with a vascular cell may be subjected to the present invention.

Further, the hydrogel may have a cell binding domain while being biodegradable. Therefore, an environment similar to the interior of a human body may be provided in the vascularized tissues. Additionally, the hydrogel may be a natural hydrogel or synthetic hydrogel.

A ratio by weight of the vascular cells to the vascularized tissue cells may range from 1 to 10. That is, increasing an amount of vascularized tissue cells higher than that of vascular cells may make it easy to identify generation of new blood vessels by a vascular endothelial growth factor (VEGF) provided from the vascularized tissue cells.

Further, after supplying the vascular cells to the supply hole, the supply hole may be filled with the hydrogel. Therefore, the same environment as the interior of the human body may be provided on the upper side of the vascular cells.

Further, before supplying the vascularized tissue cells in the container, the hydrogel may be supplied and cured therein. Therefore, the hydrogel may cover the entire surrounding of the vascularized tissue cells.

### [Advantageous Effects]

According to the method for manufacturing *in vitro* vascularized tissues of the present invention, angiogenesis of cancer cells, liver cells, nerve cells, or the like, using vascular cells *in vitro* may enable *in vitro* mass production of cancer tissues, liver tissues, brain tissues, or the like, simultaneously.

Accordingly, using the *in vitro* vascularized tissues obtained by the present invention, diseases associated with vascularized tissues may be subjected to *in vitro* research and a treatment method of the diseases may be developed. Further, an appropriate method for treating individual patients can be quickly determined.

Therefore, according to the present invention, an effective treatment rate of a disease associated with vascularized tissues such as cancer, liver disease, stroke, dementia, or the like, may increase due to existing anticancer medicines or treatment methods.

Specifically, for radiation-based tumor or cancer treatment, the present invention may also be applied to *in vitro* mass production of cancer tissues, thereby determining a type and dosage of radiation suitable for removal of corresponding cancer cells. As a result, even in radiation-based cancer treatment, side effects caused by incorrect use of radiation may be reduced to thus remarkably improve a cancer treatment rate.

### [Description of Drawings]

FIG. 1 is a flow diagram illustrating a method for manufacturing *in vitro* vascularized tissues according to the present invention.
FIG. 2 is a view illustrating a drug experiment using *in vitro* vascularized tissues formed by the method for manufacturing *in vitro* vascularized tissues according to the present invention.
FIG. 3 is a view illustrating a modified embodiment of the method for manufacturing *in vitro* vascularized tissues according to the present invention, as shown in FIG. 1.

### [Best Mode]

Hereinafter, preferable embodiments of the present invention will be described with reference to the accompanying drawings. Referring to the drawings, like reference characters designate like or corresponding parts throughout the several views. In the embodiments of the present invention, a detailed description of publicly known functions and configurations that are judged to be able to make the purpose of the present invention unnecessarily obscure are omitted.

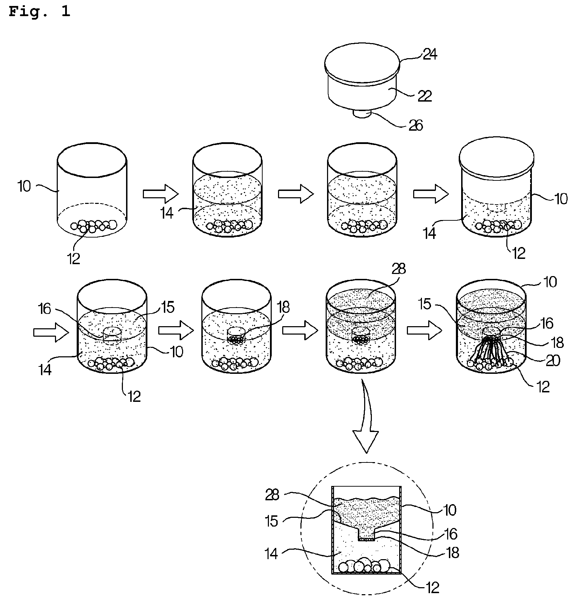

FIG. 1 illustrates a method for manufacturing *in vitro* vascularized tissues according to the present invention.

First, vascularized tissue cells 12 are supplied in a container 10. The container 10 may consist of a transparent material such as glass or plastic in order to observe an inside thereof and shapes of the container is not particularly limited.

The vascularized tissue cells 12 may be selected from cancer cells, brain cells and liver cells, and the vascularized tissue cells 12 may be taken from a patient. For instance, if cancer cells are used, approximately 20,000 to 40,000 cells may be provided. The vascularized tissue cells are cultured in a cell culture solution and, after removing the cell culture solution, the cultured tissue cells may be supplied in the container 10. The cell culture solution may be any conventional cell culture fluid and, for example, Dulbecco's Modified Eagle's Medium (DMEM) or α-Minimum Essential Medium (α-MEM) may be used.

Further, the hydrogel 14 is supplied over the vascularized tissue cells 12. The hydrogel 14 must have a cell binding domain while being biodegradable. The foregoing hydrogel 14 may be a natural hydrogel or synthetic hydrogel. The natural hydrogel may include collagen or the like, while the synthetic hydrogel may include, for example, commercial QGel (QGel™, QGel SA, Switzerland). Using the foregoing hydrogel 14, a moisture-containing environment such as that in the interior of a human body may be provided to the vascularized tissue cells 12.

Alternatively, before supplying the vascularized tissue cells 12, the hydrogel 14 may be supplied in the container 10 and cured therein. Accordingly, the hydrogel 14 may thoroughly surround the vascularized tissue cells 12 to provide an environment similar to the interior conditions of the human body.

Further, a supplier may be formed on top of the hydrogel 14. The supplier should have a shape of gathering blood cells 18 in a specific position. Also, the supplier may have a dent 15 formed to be inclined or curved by lowering a center part further than a peripheral part of the dent, or a supply hole 16 may be formed by cutting a groove at a specific site. According to an embodiment of the present invention, as shown in FIG. 1, such a dent 15 as described above is formed and, at the same time, the supply hole 16 is provided in the center of the dent 15. The supplier should be separated from the vascularized tissue cells 12, therefore, must be spaced apart from the vascularized tissue cells 12, as shown in FIG. 1.

In order to form the supplier as described above, the present invention uses a molding cover 22. The molding cover 22 is formed to cover the container 10 wherein a sloping side corresponding to the dent 15 is provided at the bottom and a molding cover protrusion 26 corresponding to the supply hole 16 is provided at the center of the sloping side. Further, applying pressure to the other part except for the supply hole 16 causes the top of the hydrogel 14 to have the same shape as the bottom of the molding cover 22.

Under these conditions, the container 10 is left at a temperature of about 35 to 40°C in an environment of 3 to 10 wt.% of carbon dioxide for 20 to 40 minutes as it is, thus curing the hydrogel 14. According to the above process, the hydrogel 14 may then retain its original shape while having the dent 15 and supply hole 16 formed therein.

Further, vascular cells 18 are supplied to the supply hole 16. Herein, the blood cells contain the vascular cells 18 and vascularized tissue cells 12 in a ratio by weight of 1 to 10. Therefore, as shown in FIG. 1, the vascularized tissue cells 12 are spread over a broader area than the supply hole 16, and the vascular cells 18 are placed on the bottom of the supply hole 16 having a smaller cross-section than that of the vascularized tissue cells 12.

Further, the cell culture solution 28 is injected on an upper side of the vascular cells 18 while supplying the vascular cells 18 to the supply hole 16. The cell culture solution 28 may be any conventional cell culture fluid and, for example, Dulbecco's Modified Eagle's Medium (DMEM) or α-Minimum Essential Medium (α-MEM) may be used.

Accordingly, through a signal material for angiogenesis generated from the vascularized tissue cells 12, new blood vessels 20 may be generated from the vascular cells 18 toward the vascularized tissues cells 12. In this regard, formation of the new blood vessels 20 that become narrow upwardly, approximately in a mountain form, can be observed in FIG. 1. As a result, the new blood vessels 20 may be clearly shown and destruction of the new blood vessels 20 may be easily observed during chemical reagent examination.

After completing the foregoing processes then passing a predetermined period of time, the new blood vessels 20 are generated from the vascular cells 18 by a signal material generated from the vascularized tissue cells 12, so as to provide *in vitro* vascularized tissues which are formed by binding the vascular cells 18 and vascularized tissue cells 12 through the new blood vessels 20.

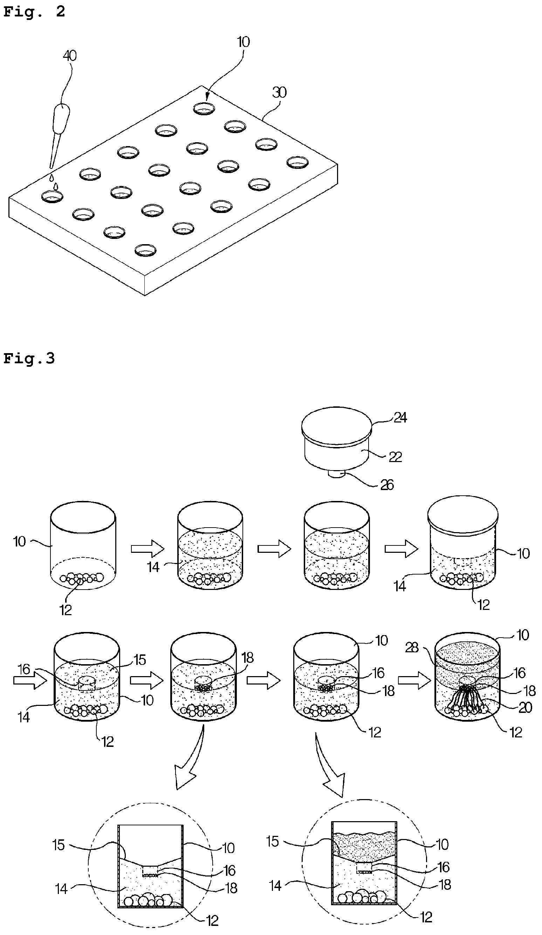

With regard to the obtained vascularized tissues, in order to conduct a variety of examinations such as drug testing or the like, a number of vascularized tissues are simultaneously manufactured on a test panel 30, as shown in FIG. 2. That is, after mounting the container 10 on the test panel 30, vascularized tissues are formed in the containers 10, respectively, by the foregoing processes. Accordingly, the vascularized tissues can be manufactured *in vitro* in large quantities.

Further, destruction of the new blood vessels 20 or vascularized tissue cells 12 is observed while varying types or dosages of known chemical reagents introduced into each of the containers 10 by a chemical injector 40. Based on the observed results, it is possible to determine an appropriate treatment method or type of chemical reagent for a patient.

FIG. 3 illustrates a modified embodiment of the method for manufacturing *in vitro* vascularized tissues according to the present invention. According to the modified embodiment, unlike the method for manufacturing *in vitro* vascularized tissues shown in FIG. 1, the vascular cells 18 are firstly supplied to the supply hole 16, followed by further injecting the hydrogel into the supply hole 16, so as to provide an environment similar to the interior of a human body in an upper space of the vascular cells 18. Other configurations are substantially the same in both of the manufacturing methods shown in FIG. 1 and FIG. 3. Therefore, a detailed description of the foregoing configurations will be omitted.

As described above, although preferred embodiments of the present invention have been described in the above detailed description, those skilled in the art will appreciate that various modifications and variations are possible without departing from the scope of the present invention disclosed in the description.

### [Description of Reference Numerals]

10: container, 12: vascularized tissue cell
14: hydrogel, 15: dent
16: supply hole, 18: vascular cell
20: new blood vessel, 22: molding cover
24: molding cover flange, 26: molding cover protrusion
28: cell culture solution, 30: test panel
40: chemical injector

## Claims

1. A method for manufacturing *in vitro* vascularized tissues, comprising:
supplying vascularized tissue cells in a container;
supplying a hydrogel to cover the vascularized tissue cells;
shaping the hydrogel to form a supply hole separated from the vascularized tissue cells on an upper side of the hydrogel; and
supplying the vascular cells to the supply hole.

2. The method according to claim 1, wherein the supply hole is formed by a molding cover to press the hydrogel and shape the same.

3. The method according to claim 1, wherein the vascularized tissue cells are at least one selected from cancer cells, brain cells and liver cells.

4. The method according to claim 1, wherein the hydrogel has a cell binding domain while being biodegradable.

5. The method according to claim 1, wherein the hydrogel is a natural hydrogel or synthetic hydrogel.

6. The method according to claim 1, wherein a ratio by weight of the vascular cells to vascularized tissue cells ranges from 1 to 10.

7. The method according to claim 1, further comprising filling the supply hole with the hydrogel after supplying the vascular cells to the supply hole.

8. The method according to claim 1, further comprising supplying the hydrogel and curing the same before supplying the vascularized tissue cells in the container.

## Patentansprüche

1. Verfahren zum in-vitro-Herstellen von vaskularisierten Geweben, umfassend:
Zuführen von vaskularisiertes-Gewebe-Zellen in einen Behälter;
Zuführen eines Hydrogels zum Bedecken der vaskularisiertes-Gewebe-Zellen;
Formen des Hydrogels, um eine von den vaskularisiertes-Gewebe-Zellen getrennte Zufuhröffnung an einer Oberseite des Hydrogels zu bilden; und
Zuführen der Gefäßzellen zu der Zufuhröffnung.

2. Verfahren gemäß Anspruch 1, wobei die Zufuhröffnung durch ein Abdeck-Formelement zum Drücken des Hydrogels und Formen desselben gebildet wird.

3. Verfahren gemäß Anspruch 1, wobei die vaskularisiertes-Gewebe-Zellen wenigstens eines ausgewählt aus Krebszellen, Hirnzellen und Leberzellen sind.

4. Verfahren gemäß Anspruch 1, wobei das Hydrogel eine zellbindende Domäne aufweist, wobei es bioabbaubar ist.

5. Verfahren gemäß Anspruch 1, wobei das Hydrogel ein natürliches Hydrogel oder ein synthetisches Hydrogel ist.

6. Verfahren gemäß Anspruch 1, wobei das Gewichtsverhältnis der Gefäßzellen zu den vaskularisiertes-Gewebe-Zellen im Bereich von 1 bis 10 liegt.

7. Verfahren gemäß Anspruch 1, ferner umfassend das Füllen der Zufuhröffnung mit dem Hydrogel nach dem Zuführen der Gefäßzellen zu der Zufuhröffnung.

8. Verfahren gemäß Anspruch 1, ferner umfassend das Zuführen des Hydrogels und Härten davon vor dem Zuführen der vaskularisiertes-Gewebe-Zellen in den Behälter.

## Revendications

1. Procédé de fabrication de tissus vascularisés *in vitro,* comprenant :
la fourniture de cellules de tissu vascularisé dans un récipient ;
la fourniture d'un hydrogel pour recouvrir les cellules de tissu vascularisé ;
le formage de l'hydrogel pour former un trou d'alimentation séparé des cellules de tissu vascularisé sur un côté supérieur de l'hydrogel ; et
la fourniture des cellules vasculaires dans le trou d'alimentation.

2. Procédé selon la revendication 1, dans lequel le trou d'alimentation est formé par une couverture de moulage pour presser l'hydrogel et former celui-ci.

3. Procédé selon la revendication 1, dans lequel les cellules de tissu vascularisé sont au moins l'une choisie parmi des cellules cancéreuses, des cellules cérébrales et des cellules hépatiques.

4. Procédé selon la revendication 1, dans lequel l'hydrogel a un domaine de liaison cellulaire tout en étant biodégradable.

5. Procédé selon la revendication 1, dans lequel l'hydrogel est un hydrogel naturel ou un hydrogel synthétique.

6. Procédé selon la revendication 1, dans lequel un rapport en poids des cellules vasculaires aux cellules de tissu vascularisé est dans la plage de 1 à 10.

7. Procédé selon la revendication 1, comprenant en outre le remplissage du trou d'alimentation avec l'hydrogel après fourniture des cellules vasculaires dans le trou d'alimentation.

8. Procédé selon la revendication 1, comprenant en outre la fourniture de l'hydrogel et le durcissement de celui-ci avant fourniture des cellules de tissu vascularisé dans le récipient.
